# EUROPEAN PATENT SPECIFICATION

(11) **EP 2 786 747 B1**
(45) Date of publication and mention of the grant of the patent: **03.05.2017**
(21) Application number: 12854273.5
(22) Date of filing: 09.01.2012
(51) Int. Cl.: A61K 9/08, A61K 33/40, A61K 33/00, A61K 33/06, A61P 17/14, A61K 31/715

(54) **PROMOTING HAIR GROWTH SOLUTION CONTAINING CHLORINE DIOXIDE PREPARATION METHOD AND USE METHOD THEREOF**
HERSTELLUNGSVERFAHREN FÜR EINE CHLORDIOXIDHALTIGE LÖSUNG ZUR FÖRDERUNG DES HAARWACHSTUMS UND VERWENDUNGSVERFAHREN DAFÜR
SOLUTION DE STIMULATION DE LA CROISSANCE CAPILLAIRE COMPRENANT DU DIOXYDE DE CHLORE, PROCÉDÉ DE PRÉPARATION ET PROCÉDÉ D'UTILISATION ASSOCIÉS

(30) Priority: 01.12.2011 CN 201110392849
(43) Date of publication of application: 08.10.2014
(73) Proprietor: Liu, Xuewu, Beijing 100123 (CN)
(72) Inventor: Liu, Xuewu, Beijing 100123 (CN)
(74) Representative: Glas, Holger
(86) International application number: PCT/CN2012/000036
(87) International publication number: WO 2013/078754

(56) References cited:
- EP-A2- 0 076 159
- CN-A- 101 785 745
- CN-A- 102 137 651
- JP-A- S5 896 011
- JP-A- S58 121 206
- US-A- 3 585 147
- US-A- 5 051 252
- US-A- 5 750 108
- LI, XIN ET AL.: 'lignan: an important natural estrogen from plants' CHINA JOURNAL OF CHINESE MATERIA MEDICA vol. 31, no. 24, 31 December 2006, pages 2021 - 2022, XP055151390
- TAN, XIAOLAN ET AL.: 'Effect of Accelerant on Percutaneous Penetration of Ciclopirox Olamine in skin' CHINA PHARMACEUTICALS 1006-4931 vol. 10, no. 5, 31 May 2001, page 49, 50, XP008173474

## Description

### Field of the invention

The present application relates to a method for treating androgenic alopecia, and particularly, the present invention relates to a hair-growth-promoting solution containing chlorine dioxide as the main component and to preparation methods thereof and methods for using the same.

### Background

In general, a normal adult typically has about 100,000 hairs on average. Hair quantity of a human is relevant to hair color thereof, and hair quantities of humans having different hair colors are different from each other markedly, for example, humans having golden hairs have 140,000 hairs on average, humans having brown hairs have 110,000 hairs on average, humans having black hairs have 108,000 hairs on average, and humans having red hairs have 90,000 hairs on average.

The pilar cycle can be broken into three successive phases: the anagen phase, the catagen phase and the telogen phase. About 85% of hairs are in the anagen phase, which grow in the rate of 0.3 mm per day (1 cm per month). One hair is in the anagen phase for a period of 2 to 6 years. After the completion of the anagen phase, the hairs proceed to the catagen phase which will last for 1 to 2 weeks, and during this phase, the hair follicles shrink to one-sixths thereof in normal circumstances. After the catagen phase, the hairs proceed to the telogen phase, and during this phase, the hairs grow slowly, even to stop growing. The telogen phase will last for about 3 month, and following this, the hair follicles will develop new hairs. If old hairs are not shed, new hairs will push old hairs out. In normal circumstances, hairs in the telogen phase will be in about 10 to 15% of all hairs. If hairs cannot transit from the catagen phase to the telogen phase (this means that new hairs cannot be developed), the exhibited phenomena just is the alopecia. In terms of the ratio of 10%, it can be calculated that about 10,000 hairs are in the non-anagen phase consisting of the catagen phase and the telogen phase, and the non-anagen phase last for about 100 days. Hairs of normal humans will naturally live through the non-anagen phase, and the hair follicles will develop new hairs to push old hairs out, so that about 100 hairs will be shed daily. However, if more than about 100 hairs will shed daily, the gradual alopecia will be exhibited. By accumulating for days and months, alopecia sufferers' hairs in the catagen phase and the telogen phase will be more and more, and the hairs will be less and less.

Now, 90% or more of alopecia sufferers (including male and female) possess a characteristic, i.e., with the alopecia, the scalp will secrete abundant grease.

According to the corresponding symptom, such alopecia will be called as "alopecia seborrheica", and in the modern medicine, it is called as "androgenic alopecia". The modern medicine puts the factor of the androgenic alopecia down to influences of DHT (dihydrotestosterone). The DHT is formed from testosterone in a reaction catalyzed by the enzyme 5α-redutase (the testosterone molecule is bound with two hydrogen atoms). This is proved substantially by the modern medicine, and in general, it also can be seen from the following two basic facts: 1. humans which are inherently deficient of 5α-redutase will not take the alopecia; 2: finasteride which can be used for treating hyperplasia of prostate (also caused by DHT) can have hair developing effects.

The mechanisms of the alopecia caused by DHT are as follows: excessive DHT accumulated on the scalp will lead to swelled sebaceous gland. Thus, on one hand, the swelled sebaceous gland will compress hair follicle cells, and on the other hand, it will produce excessive grease. The compression to the hair follicle cells means that the sebaceous gland and the hair follicle cells struggle for living spaces. Non-inherent swelling of the sebaceous gland will necessarily compress the living space of the hair follicles cells. Furthermore, this will produce impacts in following two aspects: (1) the compressed hair follicles and developed hair roots thereof will likewise be squeezed; since the root portion and the exit are squeezed, grown hairs will become fine, and if the hairs become fine to an extent, the hairs will be naturally bred; thus, no new hairs will grow out; after accumulating for days and months, hairs will be less and less, just like that if top soil is compacted, underlying plantlet can not grow up; (2) if hair follicles are squeezed, blood vessel supplying blood to the hair follicles will be influenced, i.e., nutritional supplements will be insufficient, and thus hairs cannot naturally continue grow, just like that if no fertilizers, crops will not better grow up. The second aspect can be further proved by actual effects of another medicament, i.e., minoxidil. The minoxidil is initially used for expanding blood vessels, but it has a special side-effect, i.e., to cause a hirsutism. When the minoxidil is used as a medicament for treating the alopecia, just the special side-effect is utilized. Such side-effect can be understood as that: the minoxidil will lead to blood vessel expansion, which results in that amounts of blood provided to hair follicles are increased, and nutritional supplements thereto are sufficient. Thus, the hair-developing ability of the hair follicles is high to such an extent that new hairs will dash out of the squeezed exit of hair follicles to grow some fine hairs.

To sum up, the mechanisms of the androgenic alopecia are as follows: the androgen in body, testosterone, is converted into DHT in the presence of 5α-redutase (II type). For a male, due to less estrogen, the conversion is particular focused on the head top. Accumulated DHT will lead to swelled sebaceous gland (just like swelled prostate) which will force hair follicles cell and hair root exits, and thus, this leads to the two direct reasons to produce the alopecia: 1. blood supplements of hair follicles are impacted, and thus nutrients cannot reach the hair follicles, so that no healthy hairs can grows out; 2. exits of hair follicles are squeezed, and thus the hairs cannot push out from the exits, so that even if any hairs can grow out, they are some unhealthy fine hairs. After accumulating for days and months, the head top will take the alopecia. As to the hair grease, it is only an accomplished symptom and does not directly result in alopecia.

The basic route for treating the alopecia comprises: directly eliminating DHT impacts; inhibiting the action of 5α-redutase so that no DHT can be formed near the scalp; or simultaneously inhibiting the above two substances. The direct results caused by the above method is the relieve of the swelling of the sebaceous gland, thereby to provide advantageous conditions so that new hairs can be developed from hair follicles and smoothly grow up. In addition, since normal hairs shed in the non-anagen phase (the catagen phase and the telogen phase), and hairs in the non-anagen phase cannot grow any more, there are no senses to prevent such hair from sheding. Only when hair follicles are stimulated, and the initial circumstances in which the alopecia takes place are improved, so that new hairs grows out and replace old hairs, the alopecia can be radically cured.

In the existing market, both medical professionals and alopecia sufferers believe two effective medicines, i.e., minoxidil and finasteride. The two medicines both are approved by FDA in USA.

### (1) Minoxidil

The minoxidil is a vasodilator for treating hypertension, and current researches show that the minoxidil can directly stimulate the proliferation and differentiation of the epitheloid cells of hair follicles, promote the angiogenesis, increase topical blood amounts, and open potassium ion passageway, so that hair follicles can transit from the telogen phase to the anagen phase. The long-time use of the minoxidil will result in the following side effect: clinically common mild dermatitis on the scalp. It is occasionally reported that the use of the minoxidil will result in some side effects, and specific reasons to produce the side-effects are not confirmed. The side effects include irritant dermatitis (inflammation, dander and burning), nonspecific allergic reaction, allergic rhinitis, urticaria, facial swelling, irritability, shortness of breath, headache, dizziness, syncope, neuritis, dizziness, chest pain, edema, blood pressure change, pulse frequency changes and palpitation.

Although the scalp administration of the minoxidil can effectively stimulate the nutritional supply of the blood vessels, and activate hair follicle to develop new hair, the minoxidil administration does not solve the fundamental factor of alopecia, i.e., due to the presence of accumulated DHT, the sebaceous gland is rendered swelled, and the swollen sebaceous gland in turn oppress hair follicles and new hairs. Hence, the minoxidil can stimulate the development of new hair from hair follicles, but cannot completely change the environments lead to the alopecia, so that the minoxidil exert its effects slowly. In general, it is required to take more than 3 months to develop new thin hairs. In addition, the drug should be administrated continuously, and once the administration is stopped, the alopecia will continue.

### (2) finasteride

finasteride is a 4-aza steroidal compounds, which is a specific inhibitor to intracellular enzyme-II type 5α-redutase during the metabolism of testosterone into the stronger DHT, but it is invalid for type I 5α-redutase.This drug can reduce DHT near blood and scalp, and can promote growth of new hairs in the alopecia area. The long-term use of finasteride will result in the side effect of influence of sexual performance, and can produce adverse effects on the reproductive system of the next generation.

According to the mechanism of modern medicine, the finasteride is effective to develop hairs. However, from actual situations, it can be seen that though the drug will be effective, it acts the effect slowly, so that it should be administrated for a long time. In general, only after the finasteride is administrated for at least 3 months, the initial effects can be exhibited. Furthermore, the administration cannot be stopped, and if it is stopped, the alopecia will continue. In addition, the finasteride which acts the scalp will be few through oral administration. Furthermore, only with a long time administration, the desirable effects can be obtained, but this will produce some side-effects. In reality, a long-term administration of the finasteride may lead to decreased sexuality, even affect the sexual desire drops, and even affect the reproductive system of next generation. As considered in another way, if the finasteride is directly administrated to the scalp, whether nor not the corresponding effects will be better and side effects will be less? However, in the existing medical filed, there are not such operation that the finasteride is directed administrated to the scalp. It is estimated that this is relevant to the high molecular weight of the finasteride molecules (the molecular weight is 372.55), and a large molecule cannot permeate below the surface of the scalp. Just like the rhinitis, it is difficult for common anti-inflammatory drugs to reach the lesion of the rhinitis.

Just because the effective ingredient which can reach the scalp is less by oral administration of the finasteride, and the finasteride is only effective to the II-type 5α-redutase, the action of eliminating DHT is poor, so that the long time administration is required. Because of the slow effects, the long time administration is required, which in turn, will lead to the risk of side-effects.

Chlorine dioxide according to the present invention is internationally recognized as a highly-effective, broad-spectrum, safe new generation sterilization and antistaling agent, which is the most ideal substitute for chlorine containing preparations, and it has be widely applied in many developed countries in the world. Relevant organizations in America, Western Europe, Canada, Japan and other developed countries, such as USA Environmental Protection Bureau, USA Food and Drug Administration, USA Department of Agriculture all approve and recommend that the chlorine dioxide is used for the sterilization, mould proofing, and food antiseptic and preservation in food, food processing, pharmaceutical, sterilization of hospitals and public environment. WHO and FAO have classified the chlorine dioxide as the safe and highly-effective A1-grade disinfectant. In order to control the production of three harmful substances (which are carcinogenic, teratogenic, mutagenic substances), in the developed countries of Europe and America the chlorine dioxide has been widely used to replace chlorine gas to disinfect drinking water. However, the chlorine dioxide as drug has not been accepted by the market.

Currently, American patent US5750108, which relates to the use of chlorine dioxide during the development of hairs and published on May 12, 1998, discloses a method of applying a chlorine dioxide solution with the concentration of 100 to 1000 ppm on the scalp, for the hair development of androgenic alopecia sufferers. The patent mentions that chlorine dioxide can inhibit 5α-reductase, thereby eliminating DHT; chlorine dioxide can also stimulate the development of new hairs from hair follicle, and it has the action of removing scurf. However, due to a low concentration, the maximum effect of the chlorine dioxide cannot be exerted, and the hair-growing effects cannot be significantly improved. A Chinese patent application No. 201010129785.4, entitled "a shampoo containing chlorine dioxide" (the publication No. CN101785745A, published on July 28, 2010), discloses a shampoo which utilizes chlorine dioxide to remove scurf. The invention only utilizes the bactericidal effect of the chlorine dioxide.

Some academic research also shows that the chlorine dioxide can be safely used in human body, for example, Shi Laishun and Xie Zhaoren ("observations to tests for the acute toxicity and the irritation of the stable chlorine dioxide", Chinese Journal of Disinfection, vol. 16, No. 1, 1996) gives experimental conclusions: the oral LD50 of the stable chlorine dioxide on mice is > 10000 mg/kg, and thus it belongs to actually non-toxic substance; the solution containing 9.7 to 11.4 mg/L of chlorine dioxide has a 48 hour skin and eye mucous irritating score of 0, and thus it belongs to non-irritant substance. Wang li, Huang Junli and Li Baixiang ("Study to the common toxicity of chlorine dioxide and by-products thereof in water", "Chinese water supply and drainage, Vol. 17, 2001) makes a conclusion: a 276.5 mg/L ClO₂ aqueous solution, a 200 mg/ L Na₂ClO₂ and NaClO₃ aqueous solution and a ClO₂ mixed water solution with total concentration of 553 mg/ L are actually non-toxic aqueous solution; a 276.5 mg/L ClO₂ aqueous solution and a 200 mg/ L NaClO₂ and NaClO₃ aqueous solution are aqueous solutions having no significant accumulated toxicity. A series of tests on rat to the ClO₂ mixed solution do not exhibit any toxic damages.
To sum up, in order to more effectively treat alopecia, it is necessary to directly administrate drugs in the vicinity of the scalp, and the corresponding effects are more direct, with no systemic effects. In addition, an externally-used mixed solution effective to treat the alopecia should have the following important effects: (1) inhibiting 5a-reducase; (2) eliminating DHT (thereby allaying the swelling of the sebaceous gland); (3) enhancing the drug permeability (drugs must permeate into the scalp); (4) anti-inflammation and anti-dandruff; (5) increasing the nutritional supply to hair follicles (for example, the minoxidil can expand blood vessels, thereby increasing nutritional supply); (6) stimulating hair follicle regeneration (a lot of hair follicles may have no activity, the regeneration can save the hair follicles).

### Detailed description of the invention

Directed to the situation that the androgenic alopecia cannot be cured now and the fact that a single compound in market cannot effectively and quickly get rid of basic factors which cause the alopecia, the objective of the present invention is, following the concept of the combined medication, to provide a mixed solution for external use which have potentiality to effectively and quickly cure the alopecia, and the preparation thereof and methods for use the same.

The present invention uses the following technical solution to solve the technical problem:
A hair-growth-promoting solution containing chlorine dioxide, made from the following components in weight percentage: chlorine dioxide or chlorine dioxide precursor 0.15-5 wt%, dimethyl sulfoxide 0-30 wt%, lycium barbarum polysaccharides 0-20 wt%, flax lignan 0-20 wt%, sodium chloride 0.2-1 wt%, a conditioner 0.5-15 wt%, a proper amount of a pH regulator to regulate the pH value to 1.5-4.5, supplemented with deionized water to 100 wt%, characterised in that the chlorine dioxide precursor is sodium chlorite or potassium chlorite. In the aforementioned solution, the pH regulator is one or more of citric acid, acetic acid, glycolic acid, salicylic acid, boric acid, sodium dihydrogen phosphate or lactic acid, and preferably citric acid.

In the aforementioned solution, the conditioner is one or more of sodium citrate, aloe extract, or vitamin B.

A method for preparing the aforementioned hair-growth-promoting solution containing chlorine dioxide, comprises the following step: deionized water is taken in a proportional amount, and lycium barbarum polysaccharides, flax lignan, a conditioner and sodium chloride are added thereto; the resultant mixture is heated to 60-75°C, with stirring to make them be completely dissolved; then the mixture is cooled to 30°C or less, and then chlorine dioxide gas or chlorine dioxide precursor is introduced; after stirring, the pH of the mixed solution is adjusted with a pH regulator to be 1.5 to 4.5.

A method for using the aforementioned hair-growth-promoting solution containing chlorine dioxide, comprises the following steps: the hair-growth-promoting solution containing chlorine dioxide is taken out from a sealed container and placed in a glass or plastic cup; subsequently, the solution is dipped with a cotton swab and then is smeared on the alopecia area, and after waiting for 1 to 2 hours, the head is washed with water.

A hair-growth-promoting solution containing a chlorine dioxide precursor comprises the following the following individual solutions which are preserved separately, in which the solutions which are preserved separately have the following components and concentrations thereof in weight percentage:
(1) a solution comprising 1%-45% of a chlorine dioxide precursor and 0-11% of sodium chloride;
(2) a solution comprising 0.45%-20% of a pH regulator and 0-60% of dimethyl sulfoxide;
(3) a solution comprising 0.45%-20% of a pH regulator and 0-40% of lycium barbarum polysaccharide;
(4) a solution comprising 0.45%-20% of a pH regulator and 0-40% of flax lignan;
(5) a solution comprising 0.45%-20% of a pH regulator and 0-40% of a conditioner;
(6) a solution comprising 0.45%-20% of a pH regulator;
wherein prior to each use, the solutions which are preserved separately are taken in a certain same volume and mixed, and the amount of the pH regulator is selected in a manner that the pH value of the resultant mixed solution prior to the use is adjusted to be 1.5 to 4.5.

In the aforementioned solution, in addition that the solution containing a chlorine dioxide precursor should be preserved separately, other solutions comprising a pH regulator can be preserved in combination.

In the aforementioned solution, the chlorine dioxide precursor may be sodium chlorite or potassium chlorite.

In the aforementioned solution, the pH regulator may be one or more of citric acid, acetic acid, glycolic acid, salicylic acid, boric acid, sodium dihydrogen phosphate or lactic acid, and preferably citric acid.

In the aforementioned solution, the conditioner is one or more of sodium citrate, aloe extract, or vitamin B.

A method for preparing the aforementioned hair-growth-promoting solution containing a chlorine dioxide precursor comprises the following steps:
(1) adding a chlorine dioxide precursor and sodium chloride in proper amount to deionized water; heating the resultant mixture to 60-75 °C with stirring to make them to be completely dissolved; then cooling the mixture to 30 °C or less, to give the first mixed solution;
(2) adding a pH regulator and dimethyl sulfoxide in proper amount to deionized water with stirring to make them to be completely dissolved, to give the second mixed solution;
(3) adding a pH regulator and lycium barbarum in proper amount to deionized water; heating the resultant mixture to 70-85 °C with stirring to make them to be completely dissolved; then cooling the mixture to 30 °C or less, to give the third mixed solution;
(4) adding a pH regulator and flax lignan in proper amount to deionized water; heating the resultant mixture to 70-85 °C with stirring to make them to be completely dissolved; then cooling the mixture to 30 °C or less, to give the fourth mixed solution;
(5) adding a pH regulator and a conditioner in proper amount to deionized water; heating the resultant mixture to 60-75 °C with stirring to make them to be completely dissolved; then cooling the mixture to 30 °C or less, to give the fifth mixed solution;
(6) adding a pH regulator in proper amount to deionized water with stirring to make it to be completely dissolved, to give the sixth mixed solution.

A method for using the aforementioned hair-growth-promoting solution containing a chlorine dioxide precursor comprises the following steps: from individual container separately containing above mentioned individual solution, solutions in the same volume are taken respectively and placed in a glass or plastic cup with a suitable size prepared in advance; the solutions are mixed and then stand for 3 to 5 min, the obtained mixed solution is dipped with a cotton swab and then is smeared on the alopecia area, and after waiting for 1 to 2 hours, the head is washed with water

The method according to the present invention has the following advantageous and beneficial effects:
(1) The present invention introduces the safety and effective oxidation sterilization and tissue regeneration ability of chlorine dioxide into the hair-growth-promoting solution system, to remove hair loss factors, stimulate the growth of hair follicles, and synchronously accomplish anti-inflammatory and anti-dandruff effects.
(2) In the present invention, the mixed solution is administrated in the alopecia area, to rapidly and sufficiently exert the effect of the hair-growth-promoting solution and reduce possible side effects.
(3) In the present invention, a plant extract is employed to supplement the hair-growth-promoting effect of chlorine dioxide, to completely change the environment causing the alopecia, and at the same time, the following effects can be achieved: inhibiting 5α-reductase, eliminating DHT, increasing the drug permeability, anti-inflammatory and dandruff, increasing nutrient supply to hair follicles, and stimulating the regeneration of hair follicles, to make the cure of androgenic alopecia possible.
(4) The invention uses the method of combined administration to increase the permeability of drugs to the scalp, and improve the stimulation of the mixed solution to the scalp.
(5) The present invention radically changes the environment for the growth of hairs, and rapidly and effectively gets rid of factors causing the alopecia; thus, even if the mixed solution is not used for a long time, healthy hairs can grow

The invention has the aforementioned significant effects due to the following reasons: a suitable concentration of chlorine dioxide is utilized, further supplemented with substances having the permeability; then the product is administrated on scalp, to go deep into hair follicle; thus, the product can achieve the effects of inhibiting 5α-reductase, reducing DHT influences, allaying the swelling of sebaceous gland, and getting rid of main factors causing the alopecia; meantime, the antibacterial ability of chlorine dioxide is utilized to remove scurf and improve scalp surface of hair follicles; further, the mixed solution stimulates hair follicles, to promote the organization regeneration, so that new hairs can be developed; by adding the lycium barbarum polysaccharide (which can restore the activity of scalp cells, reduce serum cholesterol and triglyceride contents, and ultimately increase blood nutrient supply) and flax lignan (plant estrogens, which can inhibit DHT, prevent skin aging, increase skin thickness, and improve skin moisture), the hair-growth-promoting ability of chlorine dioxide can be supplemented under the action of medical solvents and conditioners, to reduce the stimulation and completely improve the environment causing the alopecia. The final mixed solution can achieve the ideal effect of rapidly developing hairs, so that the alopecia sufferers can be cured without a long time administration.

### Embodiments

By the following specific embodiments, the product and method according to the invention is described in a further and detailed manner.

The hair-growth-promoting solution for external use containing chlorine dioxide according to the invention can be classified into two forms: a single solution containing chlorine dioxide and a combination of several solutions containing chlorine dioxide precursors. Hence, the product according to the invention can be classified into two forms: a single solution containing chlorine dioxide and a combination of several solutions containing chlorine dioxide precursors.

A hair-growth promoting solution is made from the following components in weight percentage: chlorine dioxide or a chlorine dioxide precursor 0.15-5, dimethyl sulfoxide 0-30, lycium barbarum polysaccharides 0-20, flax lignan0-20, sodium chloride 0.2-1, a conditioner 0.5-15, a proper amount of a pH regulator to regulate the PH value to 1.5-4.5, supplemented with deionized water to 100.

The method for preparing the aforementioned single solution containing chlorine dioxide is as follow: deionized water is taken in a specific proportional amount, and lycium barbarum polysaccharide, flax lignan, conditioners and sodium chloride are added thereto; the resultant mixture is heated to 60-75°C with stirring to make them to be completely dissolved; after cooling the mixture to 30 °C or less, chlorine dioxide gas or chlorine dioxide precursors are introduced thereto; after stirring, the pH value of the mixed solution is regulated with a pH regulator to be 1.5 to 4.5, to give the product.

A method for using the aforementioned hair-growth-promoting single solution containing chlorine dioxide comprises the following steps: from a sealed container, the product is taken in a suitable amount (depending on personal situation and the size of alopecia area) and placed in a glass or plastic cup with a suitable size (the container is sealed immediately to prevent the volatilization of chlorine dioxide); the product is dipped with a cotton swab and then is smeared on the alopecia area, and after waiting for 1 to 2 hours, the head is washed with water.

Since chlorine dioxide cannot be stably held in an aqueous solution for a long time, the mixed solution according to the invention can be divided into several parts to be preserved separately. Prior to each use (prior to smearing the solution on the alopecia area), the parts are mixed and then used, which either can avoid reactions between different substances, or can avoid the excessive volatilization of chlorine dioxide. Upon using the solutions by mixing, individual solutions which are preserved separately are taken in a certain same volume (for example, the drop number) and then mixed.

Several solutions which are preserved separately have the following components and concentrations thereof in weight percentage:
(1) a solution comprising 1%-45% of a chlorine dioxide precursor and 0-11% of sodium chloride;
(2) a solution comprising 0.45%-20% of a pH regulator and 0-60% of dimethyl sulfoxide;
(3) a solution comprising 0.45%-20% of a pH regulator and 0-40% of lycium barbarum polysaccharide;
(4) a solution comprising 0.45%-20% of a pH regulator and 0-40% of flax lignan;
(5) a solution comprising 0.45%-20% of a pH regulator and 0-40% of a conditioner;
(6) a solution comprising 0.45%-20% of a pH regulator.

The amount of the pH regulator is selected in a manner that the pH value of the mixed solution (prior to each use, several solutions which are preserved separately are taken and mixed in a certain same volume) prior to the use is adjusted to be from 1.5 to 4.5.

Preferably, the several aforementioned solutions are used according to the rule that each solution which is preserved separately is taken in the same volume (such as the drop number), and individual solutions which are preserved separately have the following components and concentrations thereof in weight percentage:
(1) a solution comprising 10%-30% of a chlorine dioxide precursor (preferably sodium chlorite) and 2.5-7.5% of sodium chloride;
(2) a solution comprising 4.5%-13.5% of a pH regulator (preferably citric acid) and 0-60% of dimethyl sulfoxide;
(3) a solution comprising 4.5%-13.5% of a pH regulator (preferably citric acid) and 0-40% of lycium barbarum polysaccharide;
(4) a solution comprising 4.5%-13.5% of a pH regulator (preferably citric acid) and 0-40% of flax lignan;
(5) a solution comprising 4.5%-13.5% of a pH regulator (preferably citric acid) and 0-40% of a conditioner;
(6) a solution comprising 4.5%-13.5% of a pH regulator (preferably citric acid).

The amount of the pH regulator (preferably citric acid) is selected in a manner that the pH value of the mixed solution (prior to each use, several solutions which are preserved separately are taken and mixed in the same volume) prior to the use is regulated to be from 1.5 to 4.5.

More preferably, the aforementioned different solutions are used according to the rule that each solution which is preserved separately is taken in the same volume (such as the drop number), and individual solutions which are preserved separately have the following components and concentrations thereof in weight percentage:
(1) a solution comprising 15%-25% of a chlorine dioxide precursor (preferably sodium chlorite) and 3.5-6% of sodium chloride;
(2) a solution comprising 6.75%-11.25% of a pH regulator (preferably citric acid) and 0-60% of dimethyl sulfoxide ;
(3) a solution comprising 6.75%-11.25% of a pH regulator (preferably citric acid) and 0-40% of lycium barbarum polysaccharide;
(4) a solution comprising 6.75%-11.25% of a pH regulator (preferably citric acid) and 0-40% and 0-40% of flax lignan;
(5) a solution comprising 6.75%-11.25% of a pH regulator (preferably citric acid) and 0-40% and 0-40% of a conditioner;
(6) a solution comprising 6.75%-11.25% of a pH regulator (preferably citric acid) and 0-40%.

The amount of the pH regulator (preferably citric acid) is selected in a manner that the pH value of the mixed solution (prior to each use, several solutions which are preserved separately are taken and mixed in the same volume) prior to the use is adjusted to be from 1.5 to 4.5.

The method for preparing the combination of several solutions containing a chlorine dioxide precursor comprises the following steps:
(1) adding a chlorine dioxide precursor and sodium chloride in proper amount to deionized water; heating the resultant mixture to 60-75°C with stirring to make them to be completely dissolved; then cooling the mixture to 30°C or less, to give the first mixed solution;
(2) adding a pH regulator and dimethyl sulfoxide in proper amount to deionized water with stirring to make them to be completely dissolved, to give the second mixed solution;
(3) adding a pH regulator and lycium barbarum in proper amount to deionized water; heating the resultant mixture to 70-85°C with stirring to make them to be completely dissolved; then cooling the mixture to 30°C or less, to give the third mixed solution;
(4) adding a pH regulator and flax lignan in proper amount to deionized water; heating the resultant mixture to 70-85°C with stirring to make them to be completely dissolved; then cooling the mixture to 30°C or less, to give the fourth mixed solution;
(5) adding a pH regulator and a conditioner in proper amount to deionized water; heating the resultant mixture to 60-75°C with stirring to make them to be completely dissolved; then cooling the mixture to 30°C or less, to give the fifth mixed solution;
(6) adding a pH regulator in proper amount to deionized water with stirring to make it to be completely dissolved, to give the sixth mixed solution.

The amount of the pH regulator is selected in a manner that the pH value of the mixed solution (prior to each use, several solutions which are preserved separately are taken and mixed in the same volume) is adjusted to be from 1.5 to 4.5.

A method for using the combination of several solutions containing a chlorine dioxide precursor comprises the following steps: from containers containing different solutions, the solutions are taken in the same volume (such as the same drop number, the total amount depends on personal situation and the size of alopecia area) and placed (dropped) in a glass or plastic cup with a suitable size prepared in advance; after the solutions are mixed and stand for 3 to 5 minutes, the obtained mixture is dipped with a cotton swab and then is smeared on the alopecia area, and after waiting for 1 to 2 hours, the head is washed with water.

There are 43 male alopecia sufferers (ages: from 22 to 68) which use the product of the invention as volunteers, and the corresponding situations are shown in Table 1.

**Table 1**

| Persons number observing effects | Effective ratio | Effect descriptions | Theoretical explanations |
|---|---|---|---|
| 42 | 98% | After 3 to 5 day upon the administration, new hairs grown on the alopecia area, like stubbles when feeling with hands and visible to naked eyes, and the new hairs are tough healthy hairs | Due to the administration on the scalp, the effect of the product is exerted quickly. The hair-growth-promoting solution can completely remove factors causing the hair loss, stimulate the hair follicles, and allay the swelling of sebaceous gland, so that new hairs can smoothly grow. |
| 43 | 100% | After 2 to 6 upon the administration, hairs on the alopecia area rapidly shed, in which fine hairs firstly shed. The more serious the alopecia is, the more the hair loss is. For the 4+ grade alopecia sufferer, after six days, all hairs in the initial alopecia area shed completely. | Among the remaining hairs in the alopecia area, the proportion of hairs in the catagen phase and the telogen phase are higher, and with the alopecia is more serous, the proportion will be gradually near to 100%; after the use of the hair-growth-promoting solution, the hair follicles develop new healthy hairs, to push out hairs in the catagen phase and the telogen phase, and the exhibited phenomena is that the hairs shed more rapidly. |
| 40 | 93% | After 15 to 25 days upon the administration (once each day), new hairs visible to naked eyes grow up; For mild alopecia sufferer, hair lines obviously move forward. | As to some of hair follicles, the stimulating effect of the hair-growth-promoting solution will lag for several days, and the exhibited phenomena is that hair follicles which do not contain hairs initially develop first hairs; under the action of the hair-growth-promoting solution, some hair follicles develop second hairs. |
| 35 | 81% | After first course of treatment with the administration, it can be judged by naked eyes that hairs restore to the next grade, for example, 2-grade hairs restore to 1-grade hairs, 4-grade hairs restore to 3-grade hairs. After 1 to 2 months, the second course of the treatment begins, and it can be judged by naked eyes that hairs restore to further next grade. | First course of treatment will cost 15 to 25 days, and after the course, new hairs grow up. During the withdraw of the drug, if no new hairs are developed in 10 days (judged by hands or naked eyes), the second course of the treatment begins, and the second course will cost 10 to 15 days, and the like |

The following examples are used for illustrate the invention, and but not used for limit the scope of the invention.

Example 1 to 7 use single solutions containing chlorine dioxide, and their components are shown in Tables 2 to 8:

**Table 2**

| Component | Amount (wt%) |
|---|---|
| sodium chlorite | 3.7 |
| sodium chloride | 0.8 |
| dimethyl sulfoxide | 0 |
| lycium barbarum polysaccharide | 0 |
| flax lignan | 0 |
| aloe extract | 0 |
| citric acid | 8.3 |
| deionized water | 87.2 |

**Table 3**

| Component | Amount (wt%) |
|---|---|
| chlorine dioxide | 0.2 |
| sodium chloride | 0.2 |
| dimethyl sulfoxide | 20 |
| lycium barbarum polysaccharide | 10 |
| flax lignan | 10 |
| Sodium citrate | 0.3 |
| aloe extract | 10 |
| citric acid | 2 |
| deionized water | 47.3 |

**Table 4**

| Component | Amount (wt%) |
|---|---|
| sodium chlorite | 4 |
| sodium chloride | 0.85 |
| dimethyl sulfoxide | 5 |
| lycium barbarum polysaccharide | 10 |
| flax lignan | 10 |
| aloe extract | 15 |
| citric acid | 9 |
| deionized water | 46.15 |

**Table 5**

| Component | Amount (wt%) |
|---|---|
| chlorine dioxide | 0.15 |
| sodium chloride | 0.2 |
| dimethyl sulfoxide | 25 |
| lycium barbarum polysaccharide | 10 |
| flax lignan | 10 |
| Sodium citrate | 0.5 |
| aloe extract | 5 |
| citric acid | 2 |
| deionized water | 47.15 |

**Table 6**

| Component | Amount (wt%) |
|---|---|
| calcium chlorite | 5 |
| sodium chloride | 1 |
| dimethyl sulfoxide | 0 |
| lycium barbarum polysaccharide | 10 |
| flax lignan | 10 |
| aloe extract | 15 |
| acetic acid | 11 |
| deionized water | 48 |

**Table 7**

| Component | Amount (wt%) |
|---|---|
| chlorine dioxide | 0.1 |
| sodium chloride | 0.1 |
| dimethyl sulfoxide | 20 |
| lycium barbarum polysaccharide | 10 |
| flax lignan | 10 |
| Sodium citrate | 0.15 |
| aloe extract | 10 |
| Sal icyl ic acid | 2 |
| deionized water | 47.65 |

**Table 8**

| Component | Amount (wt%) |
|---|---|
| barium chlorite | 7.5 |
| sodium chloride | 1.5 |
| dimethyl sulfoxide | 0 |
| lycium barbarum polysaccharide | 10 |
| flax lignan | 10 |
| aloe extract and vitamin B | 20 |
| sodium dihydrogen phosphate | 12 |
| deionized water | 39 |

Example 8 and Example 14 both relate to several solutions containing a chlorine dioxide precursor, and their formulas are shown in Table 9-Table 15:

**Table 9**

| solutions preserved separately | Component | Amount (wt%) | Volume ratio (vol) |
|---|---|---|---|
| the first solution | sodium chlorite | 20 | 100 |
| | sodium chloride | 4 | |
| | deionized water | 76 | |
| the second solution the third solution the fourth solution | citric acid | 45 | 500 |
| | lycium barbarum polysaccharide | 0 | |
| the fifth solution the sixth solution preserved in combination | flax lignan | 0 | |
| | aloe extract | 0 | |
| | dimethyl sulfoxide | 0 | |
| | deionized water | 455 | |

**Table 10**

| Solutions preserved separately | Component | Amount (wt%) | Volume ratio (vol) |
|---|---|---|---|
| the first solution | sodium chlorite | 15 | 100 |
| | sodium chloride | 3.5 | |
| | deionized water | 81.5 | |
| the second solution | citric acid | 6.75 | 100 |
| | lycium barbarum polysaccharide | 10 | |
| | deionized water | 83.25 | |
| the third solution | citric acid | 6.75 | 100 |
| | flax lignan | 10 | |
| | deionized water | 83.25 | |
| the fourth solution | citric acid | 6.75 | 100 |
| | aloe extract | 10 | |
| | deionized water | 83.25 | |
| the fifth solution | citric acid | 6.75 | 100 |
| | dimethyl sulfoxide | 15 | |
| | deionized water | 78.25 | |
| the sixth solution | citric acid | 6.75 | 100 |
| | deionized water | 93.25 | |

**Table 11**

| Solutions preserved separately | Component | Amount (wt%) | Volume ratio (vol) |
|---|---|---|---|
| the first solution | sodium chlorite | 25 | 100 |
| | sodium chloride | 5 | |
| | deionized water | 70 | |
| the second solution | citric acid | 11 | 100 |
| | lycium barbarum polysaccharide | 20 | |
| | deionized water | 69 | |
| the third solution | citric acid | 11 | 100 |
| | flax lignan | 20 | |
| | deionized water | 69 | |
| the fourth solution | citric acid | 11 | 100 |
| | aloe extract | 20 | |
| | deionized water | 69 | |
| the fifth solution | citric acid | 11 | 100 |
| | dimethyl sulfoxide | 5 | |
| | deionized water | 84 | |
| the sixth solution | citric acid | 11 | 100 |
| | deionized water | 89 | |

**Table 12**

| Solutions preserved separately | Component | Amount (wt%) | Volume ratio (vol) |
|---|---|---|---|
| the first solution | sodium chlorite | 10 | 100 |
| | sodium chloride | 2.5 | |
| | deionized water | 87.5 | |
| the second solution | citric acid | 4.5 | 100 |
| | lycium barbarum polysaccharide | 10 | |
| | deionized water | 85.5 | |
| the third solution | citric acid | 4.5 | 100 |
| | flax lignan | 10 | |
| | deionized water | 85.5 | |
| the fourth solution | citric acid | 4.5 | 100 |
| | aloe extract | 10 | |
| | deionized water | 85.5 | |
| the fifth solution | citric acid | 4.5 | 100 |
| | dimethyl sulfoxide | 20 | |
| | deionized water | 75.5 | |
| the sixth solution | citric acid | 4.5 | 100 |
| | deionized water | 95.5 | |

**Table 13**

| Solutions preserved separately | Component | Amount (wt%) | volume ratio (vol) |
|---|---|---|---|
| the first solution | sodium chlorite | 30 | 100 |
| | sodium chloride | 7 | |
| | deionized water | 63 | |
| the second solution | citric acid | 13.5 | 100 |
| | lycium barbarum polysaccharide | 10 | |
| | deionized water | 76.5 | |
| the third solution | citric acid | 13.5 | 100 |
| | flax lignan | 10 | |
| | deionized water | 76.5 | |
| the fourth solution | citric acid | 13.5 | 100 |
| | aloe extract | 20 | |
| | deionized water | 66.5 | |
| the fifth solution | citric acid | 13.5 | 100 |
| | dimethyl sulfoxide | 0 | |
| | deionized water | 86.5 | |
| the sixth solution | citric acid | 13.5 | 100 |
| | deionized water | 86.5 | |

**Table 14**

| Solutions preserved separately | Component | Amount (wt%) | Volume ratio (vol) |
|---|---|---|---|
| the first solution | potassium chlorite | 1 | 100 |
| | sodium chloride | 0.25 | |
| | deionized water | 98.75 | |
| the second solution | sodium dihydrogen phosphate | 1 | 100 |
| | lycium barbarum polysaccharide | 10 | |
| | deionized water | 89 | |
| the third solution | sodium dihydrogen phosphate | 1 | 100 |
| | flax lignan | 10 | |
| | deionized water | 89 | |
| the fourth solution | sodium dihydrogen phosphate | 1 | 100 |
| | aloe extract | 5 | |
| | deionized water | 94 | |
| the fifth solution | sodium dihydrogen phosphate | 1 | 100 |
| | dimethyl sulfoxide | 30 | |
| | deionized water | 69 | |
| the sixth solution | sodium dihydrogen phosphate | 1 | 100 |
| | deionized water | 99 | |

**Table 15**

| Solutions preserved separately | Component | Amount (wt%) | Volume ratio (vol) |
|---|---|---|---|
| the first solution | Barium chlorite | 45 | 100 |
| | sodium chloride | 11 | |
| | deionized water | 44 | |
| the second solution | lactic acid | 15 | 100 |
| | lycium barbarum polysaccharide | 10 | |
| | deionized water | 75 | |
| the third solution | lactic acid | 15 | 100 |
| | flax lignan | 10 | |
| | deionized water | 75 | |
| the fourth solution | lactic acid | 15 | 100 |
| | aloe extract and vitamin B | 30 | |
| | deionized water | 55 | |
| the fifth solution | lactic acid | 15 | 100 |
| | dimethyl sulfoxide | 0 | |
| | deionized water | 85 | |
| the sixth solution | lactic acid | 15 | 100 |
| | deionized water | 85 | |

While the present invention has been described and illustrated by reference to
general descriptions and particular embodiments, on the basis of the invention, it is obvious for those of ordinary skill in the art to make some amendments or improvements. Hence, these amendments or improvements made on the basis that the spirits of the present invention are not deviated belong to the scope as claimed in the present invention.

## Claims

1. A hair-growth-promoting solution containing chlorine dioxide, made from the following components in weight percentage: chlorine dioxide or a chlorine dioxide precursor 0.15-5 wt%, dimethyl sulfoxide 0-30 wt%, lycium barbarum polysaccharides 0-20 wt%, flax lignan 0-20 wt%, sodium chloride 0.2-1wt%, a conditioner 0.5-15 wt%, a proper amount of a pH regulator to regulate the pH value to 1.5-4.5, supplemented with deionized water to 100 wt%, **characterized in that**: the chlorine dioxide precursor is sodium chlorite, or potassium chlorite; the pH regulator is one or more of citric acid, acetic acid, glycolic acid, salicylic acid, boric acid, sodium dihydrogen phosphate and lactic acid; the conditioner is one or more of sodium citrate, aloe extract or vitamin B.

2. A hair-growth-promoting solution containing chlorine dioxide according to claim 1, **characterized in that**: the amount of the chlorine dioxide or chlorine dioxide precursor ranges from 0.2 to 4 wt%, the amount of said sodium chloride ranges from 0.2 to 0.85 wt%, and the amount of said conditioner ranges from 1 to 10 wt%.

3. A method for preparing the hair-growth-promoting solution containing chlorine dioxide according to claim 1, **characterized in that** the method comprises the following steps: lycium barbarum polysaccharide, flax lignan, conditioner and sodium chloride are taken in proper ratio and added to deionized water; the resultant mixture is heated to 60-75°C with stirring to make them to be completely dissolved; after cooling the mixture to 30°C or less, chlorine dioxide gas or chlorine dioxide precursors are introduced thereto; after stirring, the pH value of the mixed solution is regulated with a pH regulator to be from 1.5 to 4.5.

4. A hair-growth-promoting solution containing a chlorine dioxide precursor, comprising the following solutions which are preserved separately, in which the solutions which are preserved separately have the following components and concentrations thereof in weight percentage:
(1) a solution comprising 1%-45% of chlorine dioxide precursor and 0-11% of sodium chloride;
(2) a solution comprising 0.45%-20% of a pH regulator and 0-60% of dimethyl sulfoxide;
(3) a solution comprising 0.45%-20% of a pH regulator and 0-40% of lycium barbarum polysaccharide;
(4) a solution comprising 0.45%-20% of a pH regulator and 0-40% of flax lignan;
(5) a solution comprising 0.45%-20% of a pH regulator and 0-40% of a conditioner;
(6) a solution comprising 0.45%-20% of a pH regulator;
**characterized in that** the chlorine dioxide precursor is sodium chlorite, or potassium chlorite; the pH regulator is one or more of citric acid, acetic acid, glycolic acid, salicylic acid, boric acid, sodium dihydrogen phosphate and lactic acid; the conditioner is one or more of sodium citrate, aloe extract or vitamin B. wherein prior to each use, the solutions which are preserved separately are taken in a certain same volume amount and mixed, and the amount of the pH regulator is selected in a manner that the pH value of the resultant mixed solution prior to the use is adjusted to be 1.5 to 4.5.

5. A hair-growth-promoting solution containing a chlorine dioxide precursor according to claim 4, **characterized in that** the solutions which are preserved separately have the following components and concentrations thereof in weight percentage:
(1) a solution comprising 10%-30% of a chlorine dioxide precursor and 2.5-7.5% of sodium chloride;
(2) a solution comprising 4.5%-13.5% of a pH regulator and 0-60% of dimethyl sulfoxide;
(3) a solution comprising 4.5%-13.5% of a pH regulator and 0-40% of lycium barbarum polysaccharide;
(4) a solution comprising 4.5%-13.5% of a pH regulator and 0-40% of flax lignan;
(5) a solution comprising 4.5%-13.5% of a pH regulator and 0-40% of a conditioner;
(6) a solution comprising 4.5%-13.5% of a pH regulator.

6. A hair-growth-promoting solution containing a chlorine dioxide precursor according to claim 5, **characterized in that** the solutions which are preserved separately have the following components and concentrations thereof in weight percentage:
(1) a solution comprising 15%-25% of a chlorine dioxide precursor and 3.5-6% of sodium chloride;
(2) a solution comprising 6.75%-11.25% of a pH regulator and 0-60% of dimethyl sulfoxide;
(3) a solution comprising 6.75%-11.25% of a pH regulator and 0-40% of lycium barbarum polysaccharide;
(4) a solution comprising 6.75%-11.25% of a pH regulator and 0-40% of flax lignan;
(5) a solution comprising 6.75%-11.25% of a pH regulator and 0-40% of a conditioner;
(6) a solution comprising 6.75%-11.25% of a pH regulator.

7. A hair-growth-promoting solution containing chlorine dioxide precursors according to claim 4, **characterized in that** the solution comprising a chlorine dioxide precursor is preserved separately, and other solutions comprising the pH regulator can be preserved in combination.

8. A hair-growth-promoting solution containing a chlorine dioxide precursor according to claim 4, **characterized in that**: the chlorine dioxide precursor is sodium chlorite, potassium chlorite, calcium chlorite, magnesium chlorite or barium chlorite; the pH regulator is one or more of citric acid, acetic acid, glycolic acid, salicylic acid, boric acid, sodium dihydrogen phosphate or lactic acid; and the conditioner is one or more of sodium citrate, aloe extract or vitamin B.

9. A method for preparing the hair-growth-promoting solution containing a chlorine dioxide precursor according to claim 4, comprises the following steps:
(1) adding a chlorine dioxide precursor and sodium chloride in proper amount to deionized water; heating the resultant mixture to 60-75°C with stirring to make them to be completely dissolved; then cooling the mixture to 30°C or less, to give the first mixed solution;
(2) adding a pH regulator and dimethyl sulfoxide in proper amount to deionized water with stirring to make them to be completely dissolved, to give the second mixed solution;
(3) adding a pH regulator and lycium barbarum polysaccharide in proper amount to deionized water; heating the resultant mixture to 70-85°C with stirring to make them to be completely dissolved; then cooling the mixture to 30°C or less, to give the third mixed solution;
(4) adding a pH regulator and flax lignan in proper amount to deionized water; heating the resultant mixture to 70-85°C with stirring to make them to be completely dissolved; then cooling the mixture to 30°C or less, to give the fourth mixed solution;
(5) adding a pH regulator and a conditioner in proper amount to deionized water; heating the resultant mixture to 60-75°C with stirring to make them to be completely dissolved; then cooling the mixture to 30°C or less, to give the fifth mixed solution;
(6) adding a pH regulator in proper amount to deionized water with stirring to make it to be completely dissolved, to give the sixth mixed solution.

## Patentansprüche

1. Eine Lösung zum Haarwuchs, in der Chlordioxid enthält, wird durch die folgenden Bestandteile mit Prozentsatz hergestellt: Chlordioxid oder Chlordioxid-Percursor 0.15-5 wt%, Dimethylsulfoxid 0-30 wt%, Bocksdorn Polysaccharid 0-20 wt%, Lignane 0-20 wt%, Natriumchlorid 0.2-1 wt%, Modifikator 0-20 wt% und dann wird angemessene PH Modifikator zur Einstellung des pH-Wertes bis 1.5-4.5 ergänzt sowie mit VE-Wasser bis 100 zugenommen, der Chlordioxid-Percusor ist Natriumchlorit oder Kaliumchlorit und das sogenannte pH-Modifiktor ist Zitronensäure, Essigsäure, Glykolsäure, Salicylsäure, Borsäure, Natriumdihydrogenphosphat und eine oder mehrere von Milchsäure; die sogenannte Konditionierungsflüssigkeit ist Natriumcitrat, Aloe-Extrakt, eine oder mehrere B-Vitamine.

2. Das Merkmal der Haarwuchslösung, die wie in Anspruch 1 beschrieben wird und Chlordioxid enthält, besteht darin, dass die Menge des Chlordioxids oder Chlordioxid-Percursor beim 0.2-4 wt% liegt, die sogenannte Menge des Natriumchlorids beträgt 0.2 bis 0.85 wt%, Die Meng des Regulierungssmittels beträgt 1-10 wt%.

3. Herstellungsverfahren u. Merkmal der Haarwuchslösung, die wie in Anspruch 1 beschrieben wird und Chlordioxid enthält, besteht darin, dass VE-Wasser verhältnismäßig genommen wird, dann wird mit LBP, Lignane, Spülungen und Natriumchlorid hingefüget und danach wird es auf 60-75 Grad Celsius erhitzt, Rühren bis es vollständig gelöst wird, dann abgekühlt auf 30 Grad Celsius oder mehr kühler, im Anschluss daran wird Chlordioxid-Percursor o. Chlordioxid zugeführt, pH - Wert der Lösung nach dem Rühren mit pH Modifikator beträgt 1.5-4.5.

4. Eine Haarwuchslösung mit Chlordioxid-Percursor enthält die folgende gemischten Lösungen, die separat gespeichert werden soll, die Bestandteile u. Konzentration der gemischten Lösungen, die separat aufbewahrt werden, mit Gewichtsprozent sind wie folgend beschrieben:
(1) die gemischte Lösung mit Chlordioxid-Percursor 1% -45%, und 0-11% Natriumchlorid;
(2) die gemischte Lösung von pH Modifikator 0.45%-20% und Dimethylsulfoxid 0-60%,
(3) die gemischte Lösung von pH Modifikator 0.45%- 20% und LBP 0-40%;
(4) die gemischte Lösung mit pH-Modifikator 0.45%- 20% und SDG 0-40% ;
(5) die gemischte Lösung mit pH-Modifikator 0.45%- 20% und Konditionierungsflüssigkeit 0-40%;
(6) die gemischte Lösung mit pH-Modifikator 0.45%- 20%,
dessen Merkmal von Chlordioxid-Precursor sind sogenannte Natriumchlorit oder Kaliumchlorit, das sogenannte pH Modifikator ist Citronensäure, Essigsäure, Glykolsäure, Salicylsäure, Borsäure, ein oder mehrere Milchsäure; das Modifikator ist Natriumcitrat, Aloe-Extrakt, eine oder mehrere B-Vitamine,
vor jeder Verwendung soll jede Lösung, die separat aufbewahrt werden, mit der gleichen Volumen gemischt werden, was die Mengewahl des pH-Modifikators angeht, sieht man die Regulierung der pH-Wert von der gemischte Lösung auf 1.5-4.5 als Ziel an.

5. Das Merkmal der Haarwuchslösung, die wie in der Anspruch 4 beschrieben wird und Chlordioxid enthält, besteht darin, dass die Bestandteile u. Konzentration der gemischten Lösungen, die separat aufbewahrt werden, mit Gewichtsprozent sind wie folgend beschrieben:
(1) die gemischte Lösung mit Chlordioxid-Percursor 10%-30% und Natriumchlorid 2.5%-7.5%;
(2) die gemischte Lösung von pH Modifikator 4.5%-13.5% und Dimethylsulfoxid 0-60%;
(3) die gemischte Lösung von pH Modifikator 4.5%- 13.5% und LBP 0-40%;
(4) die gemischte Lösung mit pH-Modifikator 4.5%-13.5%und SDG 0-40%;
(5) die gemischte Lösung mit pH-Modifikator 4.5%- 13.5% und Pflegungsmittel 0-40%;
(6) die gemischte Lösung mit pH-Modifikator 4.5%-13.5%.

6. Das Merkmal der Haarwuchslösung, die wie in der Anspruch 5 beschrieben wird und Chlordioxid enthält, besteht darin, dass die Bestandteile u. Konzentration der gemischten Lösungen, die separat aufbewahrt werden, mit Gewichtsprozent sind wie folgend beschrieben,
(1) die gemischte Lösung mit Chlordioxid-Percursor 15%-25% und Natriumchlorid 3.5%-6%;
(2) die gemischte Lösung von pH Modifikator 6.75%-11.25% und Dimethylsulfoxid 0-60%;
(3) die gemischte Lösung von pH Modifikator 6.75%-11.25% und LBP 0-40%;
(4) die gemischte Lösung mit pH-Modifikator 6.75%-11.25% und SDG 0-40%;
(5) die gemischte Lösung mit pH-Modifikator 6.75%-11.25% und Pflegungsmittel 0-40%;
(6) die gemischte Lösung mit pH-Modifikator 6.75%-11.25%.

7. Das Merkmal der Haarwuchslösung, die wie in Anspruch 4 beschrieben wird und Chlordioxid enthält, besteht darin, dass die gemischte Lösung mit Chlordioxid-Precursor separat gespeichert werden soll und die anderen gemischten Lösungen, die pH-Modifikator enthalten, zusammengespeichert werden soll.

8. Das Merkmal der Haarwuchslösung, die wie in Anspruch 4 beschrieben wird und Chlordioxid enthält, besteht darin, der Chlordioxid-Precursor ist Natriumchlorit, Kaliumchlorit, Chlorit, Calcium - Chlorit, Magnesium oder Barium Chlorit; der sogenannte pH-Modifikator ist Zitronensäure, Essigsäure, Glykolsäure, Salicylsäure, Borsäure, Natriumdihydrogenphosphat und ein oder mehrere von Milchsäure; der Konditionierungsflüssigkeit ist Natriumcitrat, Aloe-Extrakt, eine oder mehrere B-Vitamine.

9. Das Herstellungsverfahren der Haarwuchslösung, die wie in Anspruch 4 beschrieben wird und Chlordioxid -Precursor enthält, besteht aus folgenden Fortschritten:
(1) Verhältnismäßiges VE-Wasser genommen und pH-Modifikator und Chlordioxid-Precursor sowie Natriumchlorid zugegeben, Erwärmung auf 60-75 °C, Rühren bis vollständig aufgelöst, Abkühlung auf 30 Grad oder weniger, d. h. das ist die erste gemischte Lösung,
(2) Verhältnismäßiges VE-Wasser genommen und pH-Modifikator u. Dimethylsulfoxid zugegeben, Rühren bis vollständig aufgelöst, d.h. das ist die zweite gemischte Lösung,
(3) Verhältnismäßiges VE-Wasser genommen und pH-Modifikator und LBP, zugegeben, Erwärmung auf 70-85°C, Rühren bis vollständig aufgelöst, Abkühlung auf 30 Grad oder weniger, d.h. das ist die dritte gemischte Lösung,
(4) Verhältnismäßiges VE-Wasser genommen und pH-Modifikator und Lignane zugegeben, Erwärmung auf 70-85°C, Rühren bis vollständig aufgelöst, Abkühlung auf 30 Grad oder weniger, d.h. das ist die vierte gemischte Lösung,
(5) Verhältnismäßiges VE-Wasser genommen und pH-Modifikator zugegeben, Erwärmung auf 60-75°C, Rühren bis vollständig aufgelöst, Abkühlung auf 30 Grad oder weniger, d.h. das ist die fünfte gemischte Lösung,
(6) Verhältnismäßiges VE-Wasser genommen und pH-Modifikator zugegeben, Rühren bis vollständig aufgelöst, d. h. das ist die sechste gemischte Lösung.

## Revendications

1. Une solution de cheveux contenant du dioxyde de chlore, qui est composée de groupes en pourcentage en poids suivants: le dioxyde de chlore ou le précurseur de dioxyde de chlore de 0.15-5 wt%, le sulfoxyde de diméthyle de 0-30 wt%, le Lycium Barbarum Polysaccharides de 0-20 wt%, le Lignane de 0-20 wt%, le chlorure de sodium de 0.2-1 wt%, et le conditionnement de 0-20 wt%, et ajouter une certaine quantité de modulateurs de pH pour régler la valeur de pH à 1.5 - 4.5, la compléter de l'eau désionisée à 100, le précurseur du dioxyde de chlore ledit est le Chlorite de sodium ou le chlorure de potassium; le modificateur de pH ledit comprend un ou plusieurs agents de l'acide citrique, de l'acide acétique, de l'acide glycolique, de l'acide salicylique et de l'acide borique, du phosphate monosodique et de l'acide lactique; le conditionnement ledit est un ou plusieurs du citrate de sodium, de l'extrait d'aloès et des vitamines B.

2. Comme la solution des cheveux contenant du dioxyde de chlore que la revendication 1 a dit, ses caractéristiques sont que la quantité de dioxyde de chlore ou de précurseur de dioxyde de chlore ledit est de 0.2-4 wt%, la quantité de chlorure de sodium ledit est de 0.2-0.85 wt%, la quantité de l'agent de conditionnement ledit est de 1-10 wt%.

3. Un procédé de préparation de solution des cheveux contenant du dioxyde de chlore décrit dans la revendication 1, ses caractérisé sont que le procédé comprend les étapes suivantes: prend l'eau désionisée selon la proportion, ajouter le Polysaccharide de Lycium barbarum, l'adhésion de Lignane, le conditionnement et le chlorure de sodium, chauffer à 60 - 75 °C, de sorte que la totalité de son agitation se dissout, puis jusqu'à la température de 30 °C ou moins, l'introduire au gaz de dioxyde de chlore ou ajouter le précurseur de dioxyde de chlore, après mélange, avec un régulateur de pH, régler la valeur de PH de la solution de mélange à 1.5 - 4.5.

4. Une solution des cheveux comprenant du précurseur de dioxyde de chlore, qui comprend la solution mélange conservée séparément ci-dessous, calculer en pourcentage en poids, les composants et la concentration de la solution mélange conservée séparément sont de:
(1) La solution de mélange qui comprend le précurseur de dioxyde de chlore de 1%-45% et le chlorure de sodium de 0-11%;
(2) La solution de mélange qui comprend le régulateur de pH de 0.45%-20% et le sulfoxyde de diméthyle de 0-60%;
(3) La solution de mélange qui comprend le régulateur de pH de 0.45%-20% et le Lycium barbarum polysaccharide de 0 - 40%;
(4) La solution de mélange qui comprend le régulateur de pH de 0.45%-20% et le Lignane de 0-40%;
(5) La solution de mélange qui comprend le régulateur de pH de 0.45%-20% et le conditionnement de 0 -40%;
(6) La solution de modulateurs de pH de 0.45%-20%,
ses caractéristiques sont que le précurseur de dioxyde de chlore ledit est le Chlorite de sodium ou le chlorure de potassium; le régulateur de pH ledit comprend un ou plusieurs agents de l'acide citrique, de l'acide acétique, de l'acide glycolique, de l'acide salicylique, de l'acide borique, du phosphate monosodique et de l'acide lactique; le conditionnement ledit comprend un ou plusieurs agents du citrate de sodium, de l'extrait d'aloès, des vitamines B,
avant chaque utilisation, prendre un volume de dosage de même et le mélanger pour chaque solution de conservation, sélectionner la quantité de régulateur de pH de manière à régler la valeur de PH de solution mélangée avant utilisation à 1.5 - 4.5.

5. Comme la revendication 4 ledit, une solution de cheveux contenant du précurseur de dioxyde de chlore, ses caractéristiques sont que elle est calculée en pourcentage en poids, les composants et la concentration de chaque solution de mélange conservée séparément sont:
(1) La solution de mélange qui comprend le précurseur de dioxyde de chlore de 10%-30% et le chlorure de sodium de 2.5%-7.5%;
(2) La solution de mélange qui comprend le régulateur de pH de 4.5%-13.5% et le sulfoxyde de diméthyle de 0-60%;
(3) La solution de mélange qui comprend le régulateur de pH de 4.5%-13.5% et le Lycium barbarum polysaccharide de 0- 40%;
(4) La solution de mélange qui comprend le régulateur de pH de 4.5%-13.5% et le Lignane de 0 - 40%;
(5) La solution de mélange qui comprend le régulateur de pH de 4.5%-13.5% et le conditionnement de 0- 40%;
(6) La solution de modulateurs de PH de 4.5%-13.5%.

6. Comme la revendication 5 ledit, une solution de cheveux contenant du précurseur de dioxyde de chlore, ses caractéristiques sont que elle est calculée en pourcentage en poids, les composants et la concentration de chaque solution de mélange conservée séparément sont:
(1) La solution de mélange qui comprend le précurseur de dioxyde de chlore de 15%-25% et la solution de chlorure de sodium de 3.5%-6%;
(2) La solution de mélange qui comprend le régulateur de pH de 6.75%-11.25% et le sulfoxyde de diméthyle de 0-60%;
(3) La solution de mélange qui comprend le régulateur de pH de 6.75%-11.25% et Lycium barbarum polysaccharide et un 0 à 40% de matière;
(4) La solution de mélange qui comprend le régulateur de pH de 6.75%-11.25% et le Lignane de 0 - 40%;
(5) La solution de mélange qui comprend le régulateur de pH de 6.75%-11.25% et le conditionnement de 0 -40%;
(6) La solution de modulateurs de pH de 6.75%-11.25%.

7. Comme la revendication 4 ledit, une solution de cheveux contenant du précurseur de dioxyde de chlore, ses caractéristiques sont que conserver séparément la solution de mélange contenant du précurseur de dioxyde de chlore, conserver de la fusion.

8. Comme la revendication 4 ledit, une solution de cheveux contenant du précurseur de dioxyde de chlore, ses caractéristiques sont que le précurseur de dioxyde de chlore ledit est le Chlorite de sodium, le chlorure de potassium, le chlorure de calcium, le magnésium ou le chlorate de baryum de chlorite; le régulateur de pH ledit comprenant un ou plusieurs agents de l'acide citrique, de l'acide acétique, de l'acide glycolique, de l'acide salicylique et de l'acide borique, du phosphate monosodique et de l'acide lactique; le conditionnement ledit comprenant un ou plusieurs agents du citrate de sodium, de l'extrait d'aloès et des vitamines B.

9. Un procédé de préparation de solution des cheveux contenant du dioxyde de chlore décrit dans la revendication 4, comprenant les étapes suivantes:
(1) prend l'eau désionisée selon la proportion, ajouter le précurseur de dioxyde de chlore et le chlorure de sodium, chauffer à 60 - 75 °C, de sorte que la totalité de son agitation se dissout, puis jusqu'à la température de 30 °C ou moins, observer la première solution mélangée;
(2) prend l'eau désionisée selon la proportion, ajouter le régulateur de pH et le sulfoxyde de diméthyle, la mélanger de rendre totalement dissous, ce qui permet d'obtenir un deuxième mélange de solution;
(3) prend l'eau désionisée selon la proportion, ajouter le modificateur de pH et le Polysaccharides de Lycium barbarum, chauffer à 70 - 85 °C, la mélanger de rendre totalement dissous, puis jusqu'à la température de 30 °C ou moins, ce qui permet d'obtenir un troisième mélange de solution;
(4) prend l'eau désionisée selon la proportion, ajouter le régulateur de pH et le Lignane, chauffer à 70- 85 °C, la mélanger de rendre totalement dissous, puis jusqu'à la température de 30 °C ou moins, ce qui permet d'obtenir un quatrième mélange de solution;
(5) prend l'eau désionisée selon la proportion, ajouter le régulateur de pH et le conditionnement, chauffer à 60 - 75 °C, la mélanger de rendre totalement dissous, puis jusqu'à la température de 30 °C ou moins, ce qui permet d'obtenir un cinquième mélange de solution;
(6) prend l'eau désionisée selon la proportion, ajouter le régulateur de pH, la mélanger de rendre totalement dissous, ce qui permet d'obtenir un sixième mélange de solution.
